(19) Europäisches Patentamt
European Patent Office
Office européen des brevets

(11) Veröffentlichungsnummer: **0 400 586 A1**

(12) # EUROPÄISCHE PATENTANMELDUNG

(21) Anmeldenummer: **90110203.8**

(51) Int. Cl.5: **A61K 31/72**

(22) Anmeldetag: **30.05.90**

(30) Priorität: **02.06.89 DE 3917982**

(43) Veröffentlichungstag der Anmeldung: **05.12.90 Patentblatt 90/49**

(84) Benannte Vertragsstaaten: **AT BE CH DE DK ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **LaRocca, Renato V.**
**344 Oaktree Lane**
**Sterlin, VA 22170(US)**
Erfinder: **Myers, Charles E.**
**11111 Ralston Rd.**
**Rockville, MD 20852(US)**
Erfinder: **Stein, Cy**
**19869 Bazzleton Place**
**Gaithersburg, MD 20879(US)**
Erfinder: **Wellstein, Anton**
**4890 Battery Lane, Apt.311**
**Bethesda, MD 20814(US)**
Erfinder: **Czech, Jörg, Dr.**
**Höhenweg 3**
**D-3550 Marburg(DE)**
Erfinder: **Dickneite, Gerhard, Dr.**
**Zum Neuen Hieb 31**
**D-3550 Marburg(DE)**
Erfinder: **Sedlacek, Hans-Harald, Dr.**
**Sonnenhang 3**
**D-3550 Marburg(DE)**
Erfinder: **Schrinner, Elmar, Dr.**
**Hedwigstrasse 3**
**D-6200 Wiesbaden(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Verwendung von Xylanpolyhydrogensulfaten zur Therapie von zellproliferations-bedingten Erkrankungen.**

(57) Es wird die Verwendung von Verbindungen der Formel I beschrieben

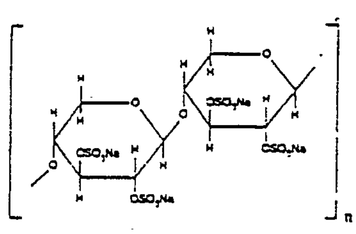

I

in der n eine ganze Zahl von 1 bis 20, vorzugsweise 8 bis 10, bevorzugt 9 ist, beziehungsweise von Gemischen mit einem mittleren Molekulargewicht von ca. 1.000 bis 20.000 Dalton, vorzugsweise von ca. 5.000 bis 12.000 Dalton, bevorzugt ca. 6.000 Dalton (n = 9), oder deren pharmakologisch unbedenkliche Säureadditionssalze zur Herstellung eines Arzneimittel zur Therapie von Erkrankungen, die durch unreguliertes und undifferenziertes Zellwachstum bedingt sind.

EP 0 400 586 A1

## Verwendung von Xylanpolyhydrogensulfaten zur Therapie von zellproliferations-bedingten Erkrankungen

Die Erfindung betrifft die Verwendung von Xylanpolyhydrogensulfaten zur Herstellung eines Arzneimittels zur Therapie von Erkrankungen, die durch unreguliertes und undifferenziertes Zellwachstum bedingt sind.

Xylanpolyhydrogensulfate sind aus DE-A-36 01 136 bekannt. Dort ist für diese Verbindungen folgende Formel I angegeben:

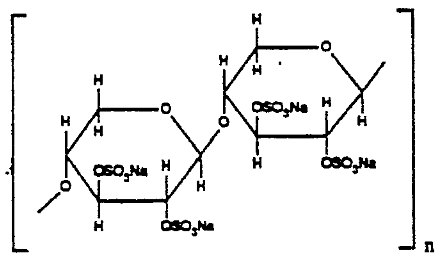

I

Ebenfalls dort ist eine anti-retrovirale Wirkung für diese Verbindungen beschrieben. Weiter ist bekannt, daß Xylanpolyhydrogensulfat die Bindung von bFGF (basic Fibroblast Growth Factor) an eine Nebennierenrindenkarzinomzellinie (SW 13) inhibiert (A. Wellstein et al., Proc. of the American Association for Cancer Research (1989) 30, 583).

Überraschenderweise wurde nun gefunden, daß Verbindungen der Formel I onkogen-kodierte Kinasen und Wachstumsfak torrezeptortyrosinkinasen inhibieren. Sie eignen sich somit zur Bekämpfung von Krankheiten, bei denen Wachstumsfaktorrezeptoren eine Rolle spielen, insbesondere Psoriasis und Tumorerkrankungen.

Die Expression von Onkogenen in einer Säugetierzelle geht mit dem Übergang vom normalen zum transformierten Zelltypus, welcher dann zu einer Krebszelle wird, einher. Die Transformation kann durch Infektion einer Zelle mit einem Retrovirus hervorgerufen werden. Ein gut bekanntes Beispiel ist die Infektion von Hühnern mit Rous-Sarkom-Virus, welche anschließend Krebs entwickeln. Das entsprechende Onkogen, das für die bösartige Transformation verantwortlich ist, wurde mit "SRC"-(Sarcom) Gen bezeichnet. Viele bis jetzt bekannte Onkogene sind durch die Expression eines Proteins mit Kinaseaktivität gekennzeichnet. Die Enzyme katalysieren die Übertragung der endständigen Phosphatgruppe des ATP auf eine Aminosäure. Im Gegensatz zu vielen anderen Proteinkinasen, die die Phosphatgruppe auf einen Seryl- oder Threonylrest übertragen, phosphorylieren die meisten onkogenkodierten Kinasen einen Tyrosylrest der Proteinkette. Abgesehen davon ist es bekannt, daß Produkte von Onkogenen, nämlich die des v-mos-, v-mil- und v-raf-Onkogens, Serin/Threonin-spezifische Proteinkinaseaktivität besitzen.

Tyrosinkinaseaktivität wird auch in Wachstumsfaktorrezeptoren exprimiert; neue Erkenntnisse zeigen nun, daß verschiedene Krankheiten, bei denen die Proliferation von Zellen eine Rolle spielt, z.B. Psoriasis und das Wachstum vieler Tumoren, von der Gegenwart von Wachstumsfaktoren abhängen, wie Epidermal Growth Factor (EGF), Transforming Growth Factor alpha (TGF alpha), Platelet Derived Growth Factor (PDGF) oder Fibroblast Growth Factor (basic FGF, acidic FGF). Im Anschluß an die Bindung des Wachstumsfaktors an seinen Rezeptor wird die Tyrosinkinase, welche eine Eigenkomponente des Wachstumsfaktorrezeptors ist, stimuliert.

Ein Inhibitor der Tyrosinkinase eines Wachstumsfaktorrezeptors inhibiert deshalb das Zellwachstums und damit z.B. auch das Tumorwachstum und die Tumorausbreitung. Er kann somit in der Tumortherapie, und in der Therapie aller Krankheiten, für die das Zellwachstum eine Rolle spielt (z.B. Psoriasis), eingesetzt werden.

Gegenstand der Erfindung ist deshalb die Verwendung einer Verbindung der Formel I

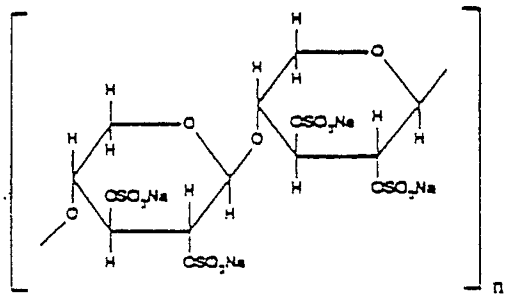

I

in der n eine ganze Zahl von 1 bis 20, vorzugsweise 8 bis 10, bevorzugt 9 ist, beziehungsweise von Gemischen mit einem mittleren Molekulargewicht von ca. 1.000 bis 20.000 Dalton, vorzugsweise von ca. 5.000 bis 12.000 Dalton, bevorzugt ca. 6.000 Dalton (n = 9), zur Herstellung eines Arzneimittels zur Inhibition von onkogen-kodierte Tyrosinkinasen, Wachstumsfaktorrezeptortyrosinkinasen und Wachstumsfaktor/-rezeptor-Wechselwirkung und damit zur Bekämpfung von zellproliferationsbedingten Erkrankungen, vorzugsweise Psoriasis, und Tumorerkrankungen.

Solche Tumoren sind besonders Karzinome und Sarkome wie Nierenkarzinom, Mammakarzinom, Prostatakarzinom, Lungenkarzinom, Ovarialkarzinom oder Rhabdomyosarkom oder Melanom.

Die erfindungsgemäßen Verbindungen besitzen vorteilhafte pharmakologische Eigenschaften, indem sie das Wachstum und die Ausbreitung von Tumoren inhibieren und können deshalb in der Tumortherapie und in der Therapie aller zellproliferationsbedingten Erkrankungen, das heißt Erkrankungen, die durch ein unreguliertes und undifferenziertes Zellwachstum gekennzeichnet sind, angewendet werden.

Zu diesem Zweck können die Verbindungen der Formel I selbst auch in Kombination oder ihre pharmakologisch unbedenklichen Säureadditionssalze verwendet werden.

Die Herstellung eines geeigneten Arzneimittels erfolgt, indem man die Wirkstoffe gegebenenfalls mit Hilfs-und/oder Trägerstoffen in eine geeignete Darreichungsform bringt.

Die folgenden Beispiele erläutern die Erfindung.

Beispiele

Test auf EGF-Rezeptortyrosinkinasehemrung

Ausgangsmaterial für die Tyrosinkinaseaktivität war die Human-Tumorzellinie A 431 (ATCC CRL 1555), die in RPMI 1640 Medium + 10 % FKS kultiviert wurde. Diese Zellinie exprimiert auf der Zelloberfläche eine große Zahl EGF-Rezeptoren, welche Tyrosinkinaseaktivität besitzen.

Die Zellen wurden bis fast zur Konfluenz kultiviert, mit PBS (Phosphate Buffered Saline, pH 7.2) gewaschen, von der Kulturflasche abgeschabt und 1 h bei 4° C inkubiert, 20x gepottert und 30′ bei 1000 g zentrifugiert. Der Überstand wurde weitere 20 min bei 20000 g zentrifugiert und das Pellet als Membranpräparation pro 1 x $10^6$ Zellen in 100 µl aufgenommen.

Die Tyrosinkinaseaktivität des EGF-Rezeptors wurde mit Poly (Glu, Ala, Tyr), (6:3:1), als Substrat gemessen. Die Zellmembranen wurden 15′ bei RT mit 1000 nM EGF vorbehandelt und dann zu dem Ansatz, der den Inhibitor, Substrat (3 mg/ml), $Mg^{2+}/Mn^{2+}$ (8 mM/ 1,6 mM), 0,16 % Triton X-100 und Natriumorthovanadat (20 µM) in 100 mM HEPES (N-2-Hydroxyethyl-Piperazin-N′-2-Ethansulfonsäure), pH 7.5, enthält, gegeben, vorinkubiert und die Reaktion durch Zugabe von gamma-$^{32}$P ATP (32 µM) begonnen. Nach 15′ bei 30° C wurde das Substrat mit 10 % TCA (Trichloressigsäure) gefällt, auf eine Millititer Filtration Plate (Millipore Corporation, Mass., USA) filtriert, gewaschen und getrocknet. Der Einbau von $^{32}$P wurde mittels eines Flüssigkeitsszintillationszählers ermittelt.

Ergebnisse:

Xylanpolyhydrogensulfat (n=9) wurde bei einer Maximalkonzentration von 1460 µg/ml geprüft und schrittweise 1:10 verdünnt. $IC_{50}$ bezeichnete die Konzentration, bei der 50 % der Ausgangsenzymaktivität gehemmt wurde. Für die EGF-Rezeptortyrosinkinase wurde ein Wert von 6 µg/ml ermittelt (Tabelle 1).

Tabelle 1

| Hemmung der enzymatischen Aktivität von Proteinkinasen durch Xylanpolyhydrogensulfat (Formel I, worin n = 9) | | |
|---|---|---|
| | $IC_{50}$ in µg/ml EGF Rezeptortyrosinkinase | $IC_{50}$ in µg/ml cAMP-abhängige Proteinkinase |
| Xylanpolyhydrogensulfat (n = 9) | 6 | 884 |

Test auf 3′,5′-cAMP-abhängige Proteinkinasehemmung

Die katalytische Untereinheit der cAMP-abhängigen Proteinkinase (Sigma) wurde, wie von Sigma (Sigma Chemical Co., St. Louis, MO, USA) beschrieben, rekonstituiert. Die Enzymaktivität wurde mit Kemptid (Sigma) (Leu-Arg-Arg-Ala-Ser-Leu-Gly) als Substrat gemessen. Der Inhibitor wurde mit Enzym, Substrat (190 µM), $Mg^{2+}$ (5 mM), 0.25 mg/ml BSA und 3.75 mM Mercaptoethanol in 50 mM MOPS (4-Morpholinpropansulfonsäure), pH 6.9, vorinkubiert. Die Reaktion wurde durch Zugabe von gamma-$^{32}P$ ATP (40 µM) begonnen. Nach 15′ bei 30° C wurde ein aliquoter Teil auf p81-Ionenaustauscher (2 x 2 cm; Whatman Paper Ltd, Großbritannien) gegeben, in 75 mM $H_3PO_4$ getaucht, gewaschen, getrocknet, und der Einbau von $^{32}P$ wurde mittels eines Flüssigkeitsszintillationszählers ermittelt.

Ergebnisse:

Xylanpolyhydrogensulfat (n = 9) wurde bei einer Maximalkonzentration von 1142 µg/ml geprüft und schrittweise 1:10 verdünnt. $IC_{50}$ bezeichnete die Konzentration, bei der 50 % der Ausgangsenzymaktivität gehemmt wurde. Die $IC_{50}$ für die cAMP-abhängige Proteinkinase betrug 884 µg/ml (Tabelle 1), d.h. Xylanpolyhydrogensulfat (n = 9) ist ein spezifischer Tyrosinkinaseinhibitor.

Inhibition des nicht adherenten Wachstums von A 549 (humane Lungenkarzinomzellinie), A 204 (humane Rhabdomyosarkomzellinie), PA-1 (humane Ovarialteratokarzinomzellinie), MDA-MB231, MDA-MB468 (humane Mammakarzinomzellinien), LnCaP (human Prostatakarzinomzellinie) und NRK (normale Rattennierenfibroblasten).

Der Test wurde entsprechend der von Hamburger und Salmon (J. Natl. Cancer Inst. 66, 981-988, 1981) beschriebenen Methodik mit den unten beschriebenen Modifikationen durchgeführt.

Konditioniertes Medium wurde durch RPMI 1640 Medium (A 549, A 204, PA-1) bzw. IMEM (alle anderen Zellinien) + 10 % FKS (Foetales Kälberserum) ersetzt. Als Folge der hohen Klonierungsrate der Tumorzellinien in Soft Agar wurde die Anzahl an Tumorzellen pro Platte bei A 549 auf 3 x $10^3$ und bei allen anderen auf 10 x $10^3$ reduziert. Die Zellen wurden in einem Zwei-Schichten-Agar System als obere Schicht entsprechend der Methode von Hamburger und Salmon (J. Natl. Cancer Inst. 66, 981-988, 1981) ausplatiert, wobei unterschiedliche Konzentrationen der Testsubstanz ± zusätzlichen EGF (Epidermal Growth Factor) (1 nM) bzw. ± zusätzlichen bFGF (basischer Fibroblast Growth Factor) (100 ng/ml) der oberen Agarschicht vor Ausplatieren der Zellen zugemischt wurden.

Die Platten wurden in einem Brutschrank mit 5% $CO_2$, 20% $O_2$ und 95 % relativer Luftfeuchte für 10 Tage (A 549 und A 204), 18 Tage (PA-1) bzw. 15 Tage (alle anderen Zellen) inkubiert. Nach dieser Zeit wurden Kolonien mit einem Durchmesser größer als 60 µm mit Hilfe eines Bildanalysegerätes gezählt. Als $IC_{50}$ wurde die jeweilige Substanzkonzentration angegeben, bei der die Anzahl der Kolonien auf die Hälfte reduziert wurde.
Der Variationskoeffizient bei wiederholten Experimenten war kleiner als 15 %.

Ergebnis:

Xylanpolyhydrogensulfat (n = 9) hemmt die Proliferation von Zellinien, die Tyrosinphosphokinase enthaltende Rezeptoren für unterschiedliche Wachstumsfaktoren aufweisen. Deshalb erfolgt eine Hemmung des

Zellwachstums, gleichgültig ob der jeweilige Wachstumsfaktor zusätzlich zugegeben wurde oder nicht (siehe 1, 2 und 4 in Tabelle 2).

Bei Zellen, welche nur in Anwesenheit eines Wachstumsfaktors (in der Zellkultur) wachsen, erfolgt gleichermaßen eine Hemmung (siehe 3). Wachstumsfaktor-unabhängige Zellen (siehe 5) werden nicht beeinflußt.

Aus der Dosis-Wirkungskurve wurde die $IC_{50}$ von Xylanpolyhydrogensulfat (n = 9) für die kontinuierliche Inkubation bestimmt. Die Ergebnisse waren:

Tabelle 2

| Tumorzellinie | | $IC_{50}$ (µg/ml) |
|---|---|---|
| 1. A 549 | | 29 (- EGF)<br>59 (+ EGF) |
| 2. PA-1 | | 4 (- bFGF)<br>6 (+ bFGF) |
| 3. A 204 | | 34 (+ bFGF)<br>(ohne bFGF keine Kolonien) |
| 4. LNcap | | 200 (- bFGF) |
| MDA 468 | EGF-abhängig | 40 |
| NRK | | 60 |
| 5. MDA 231 | | - |

Inhibition des Humantumorwachstums im Nierenkapselassay (Nacktmaus)

1. Aufarbeitung des Tumors

Die zu testenden Humantumore werden routinemäßig in nu/nu CD1-Mäusen gehalten und passagiert. Der Tumor wird unter sterilen Bedingungen entfernt, von Bindegewebe und nekrotischen Teilen befreit, zerkleinert und mit 20 ml folgender Lösung (100 ml PBS ohne $Ca^{2+}$ und $Mg^{2+}$ mit 800 mg Collagenase (Serva 0.6-0.8 U/ml) + 2 mg DNAse) aufgelöst. Inkubation: 2 h, 37° C unter Schütteln. Danach wird die Zellsuspension durch ein Nylonnetz (Porendurchmesser 51 µm) filtriert und dreimal mit $Ca^{2+}$ und $Mg^{2+}$ freiem PBS gewaschen. Danach werden die Zellen gezählt und pelletiert.

2. Einbetten der Zellen in Fibrinstücke

Die innere Wand einer 50 µl Glaskapillare (Durchmesser 1.5 mm) wird mit einer Thrombin/$CaCl_2$ Lösung (50 units Thrombin in 1 ml 40 mmol/l $CaCl_2$) benetzt.

Das Tumorzellpellet (Tumor oder Tumorzellinie) wird in einer koagulierbaren Fibrinogenlösung wie $^R$Beriplast (Behringwerke AG) suspendiert und 1:2 mit RPMI + 15 % FKS zu einer endgültigen Zellkonzentration von $10^7$ Zellen/50 µl verdünnt. Diese Suspension wird schnell in die Glaskapillaren eingesaugt.

Nach dem Verfestigen der Zell-Fibrinmischung (ca. 5 Min. bei Raumtemperatur) wird der Fibrinkleber mit Luftdruck aus den Glaskapillaren in Petrischalen gepreßt. Der Fibrinkleber wird in 2 mm Stückchen (entsprechend 5 x $10^5$ Zellen/Stück) zerschnitten, und die Stücke werden in RPMI + 15 % FKS bis zur Implantation gelagert.

3. Implantation

Die Tiere werden mit Nembutal (1:7 mit physiologischer Kochsalzlösung verdünnt; 0.1 ml/10 g Maus) betäubt. Die Niere wird durch einen 1.5 cm langen Schnitt in die Flanke freigelegt. Die Nierenkapsel wird aufgeschnitten und ein einzelnes Fibrinstück unter die Nierenkapsel transferiert. Danach werden die zwei Durchmesser des implantierten Stückes mit einem Mikroskop mit Okularmikrometer vermessen. Die Tumorgröße wird gemäß V = a x b, worin V = Tumorgröße, a = größter Tumordurchmesser und b = Tumordurchmesser senkrecht zu a sind, bestimmt.

Danach wird das Peritoneum mit Gewebekleber (Histoacryl) geschlossen und die Haut geklammert.

Behandlung

Xylanpolyhydrogensulfat (n=9) wird i.v. täglich an Tag 2-10 mit der maximal tolerablen Dosis und mit 2/3 der maximal tolerablen Dosis entsprechender Vortests verabreicht.

Messung

Am Tag 20 nach der Implantation werden die Tiere getötet, die Niere freigelegt und die Tumorgröße erneut gemessen. Die Effektivität der Testsubstanz wird durch die Tumorwachstumsinhibition bestimmt.

Die relative Tumorgröße $V_R$ wird entsprechend $V_R = V_t/V_0$ berechnet, worin $V_t$ die Tumorgröße am Ende des Experimentes (Tag 20) und $V_0$ die Tumorfibrinstückchengröße am Tag der Implantation bedeuten.

Danach wird der Median der relativen Tumorgröße der behandelten Gruppe ($V_T$) in Beziehung zum entsprechenden Median der relativen Tumorgröße der Kontrolle ($V_C$) gesetzt und T/C % = $V_T/V_C$ x 100 berechnet.

Die statistische Signifikanz des antitumoralen Effektes wird mit Hilfe des Wilcoxon U Tests bestimmt. Die relativen Tumorgrößen in der behandelten Gruppe werden mit den entsprechenden relativen Größen der Kontrollgruppe verglichen und die Änderung in der Tumorgröße wird nur als Substanz-spezifisch angesehen, wenn sie mit p kleiner als 0.05 statistisch signifikant ist.

Ergebnis:

Tabelle 3

| Tumor | Dosis | Aktivität (T/C %) |
|---|---|---|
| LXF 529 (humanes Bronchialkarzinom) | 20 mg/kg<br>30 mg/kg | 70<br>71 |
| MCF7 (humanes Mammakarzinom) | 20 mg/kg<br>30 mg/kg | 66<br>65 n.s. |
| Ovar-6 (humanes Ovarialkarzinom) | 20 mg/kg<br>30 mg/kg | 78 n.s.<br>66 |
| PaTu 8902 (humanes Pankreaskarzinom) | 20 mg/kg<br>30 mg/kg | 56<br>27 |
| HT 29 (humanes Kolonkarzinom) | 20 mg/kg<br>30 mg/kg | 71<br>76 n.s. |
| n.s. = nicht signifikant | | |

Inhibition des Humantumorwachstums in subkutan wachsenden Humantumoren (Nacktmaus)

Die getesteten Humantumore werden routinemäßig in nu/nu CD1-Mäusen gehalten und passagiert. Die Tumore werden bei jeder dritten Passage auf ihren humanen Charakter durch Immunhistologie mit monoklonalen Antikörpern geprüft. Der Tumor wird unter sterilen Bedingungen entfernt und in kleine Stücke von etwa 1-10 $mm^3$ Volumen zerschnitten. Je ein Tumorstückchen wird subkutan in die Seite einer Nacktmaus implantiert. Nach 7-14 Tagen ist das Tumorstück im umgebenden Gewebe angewachsen und die Tumorgröße wird aus den 2 Durchmessern nach folgender Formel bestimmt: V = a x b (s. vorn).

Diese Verfahren wird zweimal pro Woche wiederholt, und nur Tiere mit progressivem Tumorwachstum werden in die Kontrollgruppe und die zu behandelnde Gruppe randomisiert. Fünf Mäuse pro Gruppe werden benutzt. Beginnend am Tag der Randomisierung werden die Tiere intravenös behandelt, entsprechend dem Behandlungsschema, das unter Ergebnis angegeben ist. Zwei unterschiedliche Dosierungen für jede Substanz werden benutzt. Die gewählten Dosierungen sind identisch mit der maximal tolerablen Dosis (MTD), die für jede Testsubstanz in einem Extraexperiment mit Nacktmäusen zuvor bestimmt wurde.

Zweimal pro Woche werden die beiden Tumordurchmesser für jede Maus gemessen, und die individuelle Tumorfläche wird entsprechend der oben genannten Formel berechnet.

Auswertung:

Die relative Tumorgröße $V_R$ und T/C % werden wie zuvor berechnet.

Die statistische Signifikanz des antitumoralen Effektes wird mit dem Wilcoxon U Test bestimmt. Die relativen Tumorgrößen in der behandelten Gruppe werden mit den entsprechenden relativen Größen der Kontrollgruppe an demselben Tag des Experimentes verglichen. Die Änderung in der Tumorfläche wird nur als substanz-spezifisch angesehen, wenn sie mit p kleiner als 0.05 statistisch signifikant ist.

Ergebnis:

Tabelle 4

| Substanz | Tumor | Dosis | Schema | Aktivität (Tag 9); (T/C %) |
|---|---|---|---|---|
| Xylanpolyhydrogensulfat (n=9) | B 17 (humanes Bronchialkarzinom) | 15 mg/kg | QD9 x i.v. | 61.4 |
| | | 20 mg/kg | QD9 x i.v. | 68.9 |

## Ansprüche

1. Verwendung einer Verbindung der Formel I

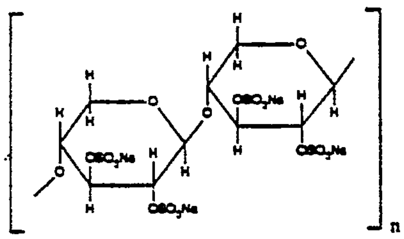

I

in der n eine ganze Zahl von 1 bis 20, vorzugsweise 8 bis 10, bevorzugt 9 ist, beziehungsweise von Gemischen mit einem mittleren Molekulargewicht von ca. 1.000 bis 20.000 Dalton, vorzugsweise von ca. 5.000 bis 12.000 Dalton, bevorzugt ca. 6.000 Dalton (n = 9), oder eines ihrer pharmakologisch unbedenklichen Säureadditionssalze zur Herstellung eines Arzneimittel zur Therapie von Erkrankungen, die durch unreguliertes und undifferenziertes Zellwachstum bedingt sind.

2. Verwendung nach Anspruch 1 zur Herstellung eines Arzneimittels zur Therapie von Karzinomen oder Sarkomen.

3. Verwendung nach Anspruch 1 zur Herstellung eines Arzneimittels zur Therapie von Nierenkarzinom, Mammakarzinom, Prostatakarzinom, Lungenkarzinom, Ovarialkarzinom, Kolonkarzinom, Pankreaskarzinom, Rhabdomyosarkom, Melanom oder der Psoriasis.

4. Verwendung einer Verbindung nach Anspruch 1 zur Herstellung eines Arzneimittels mit einer hemmenden Wirkung auf onkogene Tyrosinkinasen und/oder Wachstumsfaktorrezeptortyrosinkinasen.

Patentansprüche für folgende Vertragsstaaten: ES, GR

1. Verfahren zur Herstellung eines Arzneimittels zur Therapie von Erkrankungen, die durch unreguliertes und undifferenziertes Zellwachstum bedingt sind, dadurch gekennzeichnet, daß eine Verbindung der Formel I

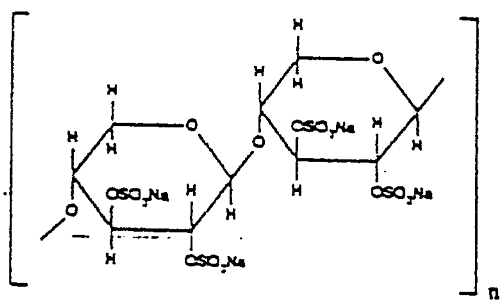

I

in der n eine ganze Zahl von 1 bis 20, vorzugsweise 8 bis 10, bevorzugt 9 ist, oder eines ihrer pharmakologisch unbedenklichen Säureadditionssalze in eine für diese Anwendung geeignete Darreichungsform gebracht wird.

2. Verfahren nach Anspruch 1, dadurch gekennzeichnet, daß ein Arzneimittel zur Therapie von Karzinomen oder Sarkomen hergestellt wird.

3. Verfahren nach Anspruch 1, dadurch gekennzeichnet, daß ein Arzneimittel zur Therapie von Nierenkarzinom, Mammakarzinom, Prostatakarzinom, Lungenkarzinom, Ovarialkarzinom, Rhabdomyosarkom, Melanom oder der Psoriasis hergestellt wird.

4. Verfahren nach Anspruch 1, dadurch gekennzeichnet, daß ein Arzneimittel mit einer hemmenden Wirkung auf onkogen-kodierte Kinasen und/oder Wachstumsfaktorrezeptortyrosinkinasen hergestellt wird.

Europäisches Patentamt

# EUROPÄISCHER RECHERCHENBERICHT

Nummer der Anmeldung

EP 90 11 0203

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | PROCEEDINGS OF THE AMERICAN ASSOCIATION FOR CANCER RESEARCH, Band 30, März 1989, Seite 583, Zusammenfassung Nr. 2320; A WELLSTEIN et al.: "Xylanpolyhydrogensulfate inhibits fibroblast growth factor dependent growth of human tumor cells" * Zusammenfassung * | 1-3 | A 61 K 31/72 |
| X | CHEMICAL ABSTRACTS, Band 92, Nr. 3, 21. Januar 1980, Seite 62, Zusammenfassung Nr. 15572m, Columbus, Ohio, US; M. KOBAYASHI et al.: "Inhibition of blood-borne pulmonary metastasis by sulfated polysaccharides", & TOKUSHIMA J. EXP. MED. 1979, 26(1-2), 41-51 * Zusammenfassung * | 1-3 | |
| X | CHEMICAL ABSTRACTS, Band 110, Nr. 5, 30. Januar 1989, Seite 31, Zusammenfassung Nr. 33511k, Columbus, Ohio, US; J.M. HERBERT et al.: "Activity of pentosan polysulfate and derived compounds on vascular endothelial cell proliferation and migration induced by acidic and basic FGF in vitro", & BIOCHEM. PHARMACOL. 1988, 37(22), 4281-8 * Zusammenfassung * | 1 | RECHERCHIERTE SACHGEBIETE (Int. Cl.5) |
| A | EP-A-0 125 152 (SANOFI) | | A 61 K<br>C 08 B |
| D,A | DE-A-3 601 136 (MAX-PLANCK GESELLSCHAFT ZUR FÖRDERUNG DER WISSENSCHAFTEN) | | |

Der vorliegende Recherchenbericht wurde für alle Patentansprüche erstellt

| Recherchenort | Abschlußdatum der Recherche | Prüfer |
|---|---|---|
| DEN HAAG | 20-07-1990 | SOMERVILLE F.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)